# EUROPEAN PATENT APPLICATION

(11) **EP 1 516 870 A1**
(43) Date of publication of application: **23.03.2005**
(21) Application number: 04022481.8
(22) Date of filing: 22.09.2004
(51) Int. Cl.: C07C 319/14, C07C 323/09, C07C 303/34, C07C 307/02, C07C 271/44, C07C 211/52, C07B 43/04, C07B 45/06

(54) **Process for producing aromatic compound derivative by reaction of aromatic compound substituted with sulfamoyloxy, sulfinamoyloxy, or carbamoyloxy group, with nucleophilic agent**

(30) Priority: 22.09.2003 JP 2003330779; 24.09.2003 JP 2003331726
(71) Applicant: Fuji Photo Film Co., Ltd., Kanagawa-ken (JP)
(72) Inventor: Takeuchi, Kiyoshi,c/oFuji Photo Film Co., Ltd., Minami-ashigara-shi Kanagawa-ken (JP); Fukunaga ,Hirofumi,c/oFuji Photo Film Co., Ltd., Minami-ashigara-shi Kanagawa-ken (JP)
(74) Representative: HOFFMANN - EITLE

(57) **Abstract**

A process for producing a compound represented by the following formula (I), which includes causing a compound represented by formula (II) to react with a nucleophilic agent: 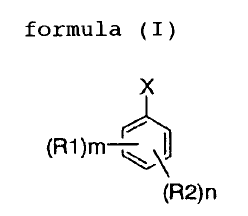 wherein X represents a substituent; R1 represents an electron-withdrawing substituent having a Hammett σₚ constant of more than 0; m represents an integer of 1 to 5; R2 represents a substituent; n represents an integer of 0 to 4; and m + n is from 1 to 5; 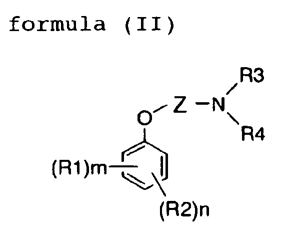 wherein R1, m, R2, and n each have the same meanings to those in formula (I); R3 and R4 each independently represent an alkyl, aryl, or heterocyclic group; and Z represents C=O or S(O)ₚ, wherein p is 1 or 2.

## Description

### FIELD OF THE INVENTION

The present invention relates to a process for synthesizing a derivative of an aromatic compound having a substituent, which is a compound useful as a synthesis intermediate of photographically-useful compounds for use in a silver halide photographic photosensitive material, medical supplies, agricultural chemicals, or others.

### BACKGROUND OF THE INVENTION

Nucleophilic reaction onto an aromatic ring of an aromatic compound is affected by the kind of group that leaves upon reaction with a nucleophilic agent (hereinafter referred to also as a "leaving group"), and the reaction is promoted by introducing an electron-withdrawing group into the aromatic ring. Examples of the leaving group include a halogen atom, an aryl- or alkylsulfonyl group, an aryl- or alkyl-sulfinyl group, an aryl- or alkyl-sulfonyloxy group, an aryl- or alkyl-thio group, an alkoxy group, an aryloxy group, an amino group, a nitro group, and an azide group. An example of the electron-withdrawing group is a nitro group.

Further, phenolic compounds, as the aromatic compound, are inexpensive and can be easily obtained. Therefore, if the hydroxyl group of the phenolic compounds can be converted to a different substituent, the conversion process would be very useful to synthesize aromatic compound derivatives. Hitherto, the following have been known: a process of converting the hydroxyl group to a p-toluenesulfonyl group, and then causing this group to react with a nucleophilic agent (see, for example, "Journal of the American Chemical Society," 1959, vol. 81, pp. 2104-2109, and "The Journal of Organic Chemistry," 2002, vol. 67, pp. 1277-1281); and a process of converting the hydroxyl group to a trifluoromethanesulfonyl group, and then causing this group to react with a nucleophilic agent (see, for example, "Synthesis," 1990, pp. 1145-1147, and "Synlett," 1999, vol. 10, pp. 1559-1562). However, the former process has the problem that the nucleophilic agent attacks the sulfur moiety of the p-toluenesulfonyl group, varyingly depending on the substituent of the aromatic compound, so as to cause a side reaction, by which the phenolic compound leaves. The latter process has a problem that trifluoromethanesulfonyl chloride or trifluoromethanesulfonic acid anhydride, which is a starting material in the process, is expensive, and thus the target compound cannot be produced at low costs.

As such, there has been a need for development of a production process that uses a phenolic compound and a starting material that can be easily obtained at low costs, to synthesize an aromatic compound derivative with high selectivity and high yield through a short process.

Further, there has been a need for a process for producing an aromatic compound derivative that is inexpensive and widely usable, at high yield through a simple process.

### SUMMARY OF THE INVENTION

The present invention is a process for producing a compound represented by the following formula (I), which includes causing a compound represented by the following formula (II) to react with a nucleophilic agent: 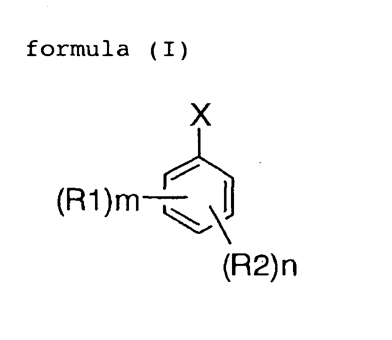 wherein X represents a substituent; R1 represents an electron-withdrawing substituent having a Hammett σₚ constant of more than 0; m represents an integer of 1 to 5 (inclusive); R2 represents a substituent; n represents an integer of 0 to 4 (inclusive); m + n is from 1 to 5; when m is 2 or more, multiple R1s may be the same or different; when n is 2 or more, multiple R2s may be the same or different; R1s, or R2s, or R1 and R2 may bond to each other to form a ring; 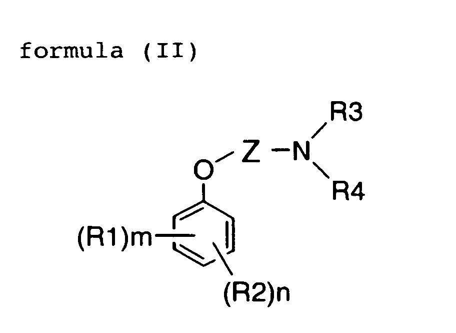 wherein R1 represents an electron-withdrawing substituent having a Hammett σₚ constant of more than 0; m represents an integer of 1 to 5 (inclusive); R2 represents a substituent; n represents an integer of 0 to 4 (inclusive); m + n is from 1 to 5 (inclusive); when m is 2 or more, multiple R1s may be the same or different; when n is 2 or more, multiple R2s may be the same or different; R1s, or R2s, or R1 and R2 may bond to each other to form a ring; R3 and R4 each independently represent an alkyl, aryl, or heterocyclic group; R3 and R4 may bond to each other to form a ring; and Z represents C=O or S(O)p, wherein p is 1 or 2.

Other and further features and advantages of the invention will appear more fully from the following description.

### DETAILED DESCRIPTION OF THE INVENTION

The inventors, having made eager investigations, have found out that the above-mentioned objects of the invention can be attained by the following process:
(1) A process for producing a compound represented by the following formula (I), which includes causing a compound represented by the following formula (II) to react with a nucleophilic agent: 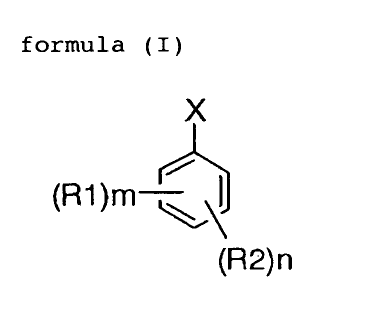 wherein X represents a substituent; R1 represents an electron-withdrawing substituent having a Hammett σₚ constant of more than 0; m represents an integer of 1 to 5 (inclusive); R2 represents a substituent; n represents an integer of 0 to 4 (inclusive); m + n is from 1 to 5 (inclusive); when m is 2 or more, multiple R1s may be the same or different; when n is 2 or more, multiple R2s may be the same or different; R1s, or R2s, or R1 and R2 may bond to each other to form a ring; 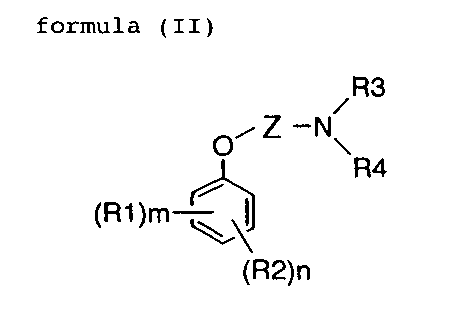 wherein R1 represents an electron-withdrawing substituent having a Hammett σₚ constant of more than 0; m represents an integer of 1 to 5 (inclusive); R2 represents a substituent; n represents an integer of 0 to 4 (inclusive); m + n is from 1 to 5 (inclusive); when m is 2 or more, multiple R1s may be the same or different; when n is 2 or more, multiple R2s may be the same or different; R1s, or R2s, or R1 and R2 may bond to each other to form a ring; R3 and R4 each independently represent an alkyl, aryl, or heterocyclic group; R3 and R4 may bond to each other to form a ring; and Z represents C=O or S(O)p, wherein p is 1 or 2.
(2) The process according to item (1), wherein the compound represented by formula (II) is a compound synthesized by causing a compound represented by the following formula (III) to react with a compound represented by the following formula (IV): 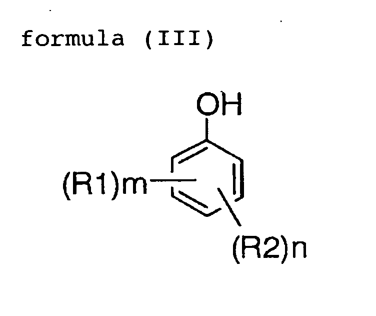 wherein R1 represents an electron-withdrawing substituent having a Hammett σₚ constant of more than 0; m represents an integer of 1 to 5 (inclusive); R2 represents a substituent; n represents an integer of 0 to 4 (inclusive); m + n is from 1 to 5 (inclusive); when m is 2 or more, multiple R1s may be the same or different; when n is 2 or more, multiple R2s may be the same or different; and R1s, or R2s, or R1 and R2 may bond to each other to form a ring; 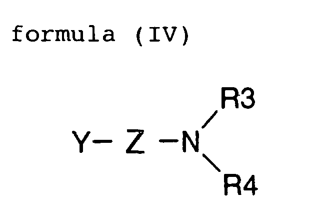 wherein R3 and R4 each independently represent an alkyl, aryl, or heterocyclic group; R3 and R4 may bond to each other to form a ring; Z represents C=O or S(O)p wherein p is 1 or 2; and Y represents a halogen atom.
(3) A process for producing a compound represented by the following formula (I), which includes causing a compound represented by the following formula (IIa) to react with a nucleophilic agent: 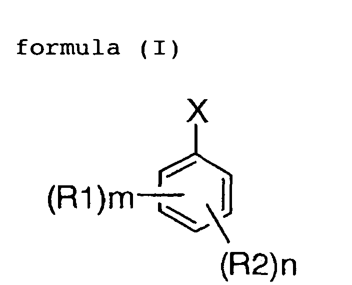 wherein X represents a substituent; R1 represents an electron-withdrawing substituent having a Hammett σₚ constant of more than 0; m represents an integer of 1 to 5 (inclusive); R2 represents a substituent; n represents an integer of 0 to 4 (inclusive); m + n is from 1 to 5 (inclusive); when m is 2 or more, multiple R1s may be the same or different; when n is 2 or more, multiple R2s may be the same or different; R1s, or R2s, or R1 and R2 may bond to each other to form a ring; 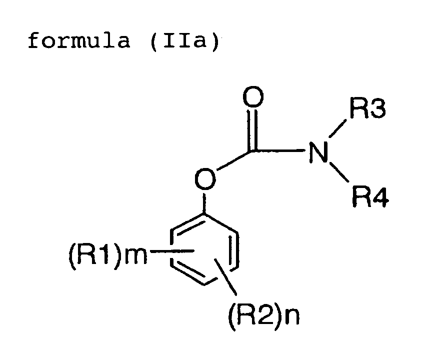 wherein R1 represents an electron-withdrawing substituent having a Hammett σₚ constant of more than 0; m represents an integer of 1 to 5 (inclusive); R2 represents a substituent; n represents an integer of 0 to 4 (inclusive); m + n is from 1 to 5 (inclusive); when m is 2 or more, multiple R1s may be the same or different; when n is 2 or more, multiple R2s may be the same or different; R1s, or R2s, or R1 and R2 may bond to each other to form a ring; R3 and R4 each independently represent an alkyl, aryl, or heterocyclic group; and R3 and R4 may bond to each other to form a ring.
(4) The process according to item (3), wherein the compound represented by formula (IIa) is a compound synthesized by causing a compound represented by the following formula (III) to react with a compound represented by the following formula (IVa): 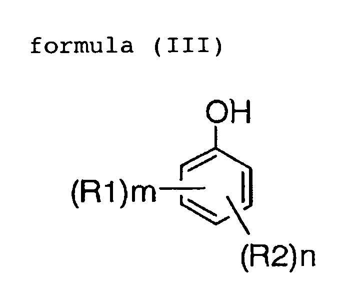 wherein R1 represents an electron-withdrawing substituent having a Hammett σₚ constant of more than 0; m represents an integer of 1 to 5 (inclusive); R2 represents a substituent; n represents an integer of 0 to 4 (inclusive); m + n is from 1 to 5 (inclusive); when m is 2 or more, multiple R1s may be the same or different; when n is 2 or more, multiple R2s may be the same or different; and R1s, or R2s, or R1 and R2 may bond to each other to form a ring; 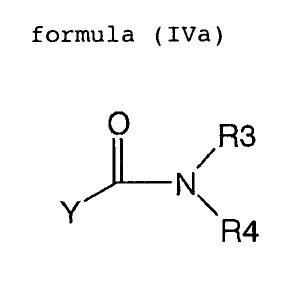 wherein R3 and R4 each independently represent an alkyl, aryl, or heterocyclic group; R3 and R4 may bond to each other to form a ring; and Y represents a halogen atom.
(5) The process according to item (3) or (4), wherein R1 is a nitro group.
(6) The process according to any one of items (3) to (5), wherein X in formula (I) is a group that bonds to the benzene ring via a nitrogen or sulfur atom.
(7) The process according to item (4), wherein the compound represented by formula (IIa) and synthesized by causing the compound represented by formula (III) to react with the compound represented by formula (IVa) is used, without being isolated, in the form of a reaction mixture.
(8) The process according to any one of items (3) to (7), wherein m in formula (I) is 1.
(9) The process according to any one of items (3) to (8), wherein R3 and R4 in formula (IIa) each are an alkyl group.
(10) The process according to the item (4), wherein Y in formula (IVa) is a chlorine atom.
   (Herein, the process for producing the compound represented by formula (I) as described in the above items (3) to (10) are collectively referred to as a first embodiment of the present invention.)
(11) A process for producing a compound represented by the following formula (I), which includes causing a compound represented by the following formula (IIb) to react with a nucleophilic agent: 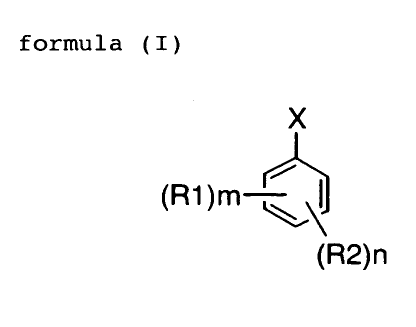 wherein X represents a substituent; R1 represents an electron-withdrawing substituent having a Hammett σₚ constant of more than 0; m represents an integer of 1 to 5 (inclusive); R2 represents a substituent; n represents an integer of 0 to 4 (inclusive); m + n is from 1 to 5 (inclusive); when m is 2 or more, multiple R1s may be the same or different; when n is 2 or more, multiple R2s may be the same or different; R1s, or R2s, or R1 and R2 may bond to each other to form a ring; 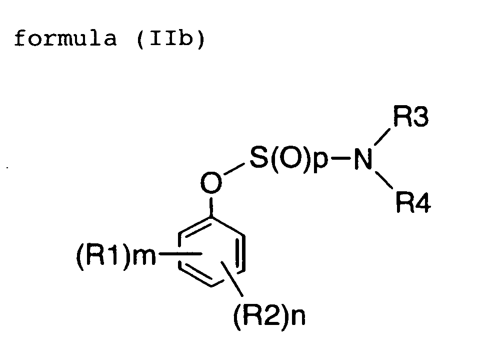 wherein R1 represents an electron-withdrawing substituent having a Hammett σₚ constant of more than 0; m represents an integer of 1 to 5 (inclusive); R2 represents a substituent; n represents an integer of 0 to 4 (inclusive); m + n is from 1 to 5 (inclusive); when m is 2 or more, multiple R1s may be the same or different; when n is 2 or more, multiple R2s may be the same or different; R1s, or R2s, or R1 and R2 may bond to each other to form a ring; R3 and R4 each independently represent an alkyl, aryl, or heterocyclic group; and R3 and R4 may bond to each other to form a ring.
(12) The process according to item (11), wherein the compound represented by formula (IIb) is a compound synthesized by causing a compound represented by the following formula (III) to react with a compound represented by the following formula (IVb): 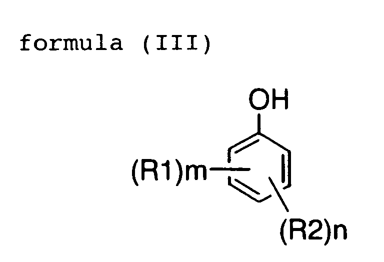 wherein R1 represents an electron-withdrawing substituent having a Hammett σₚ constant of more than 0; m represents an integer of 1 to 5 (inclusive); R2 represents a substituent; n represents an integer of 0 to 4 (inclusive); m + n is from 1 to 5 (inclusive); when m is 2 or more, multiple R1s may be the same or different; when n is 2 or more, multiple R2s may be the same or different; and R1s, or R2s, or R1 and R2 may bond to each other to form a ring; 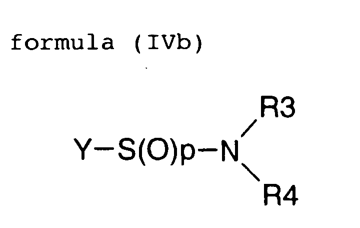 wherein R3 and R4 each independently represent an alkyl, aryl, or heterocyclic group; R3 and R4 may bond to each other to form a ring; p represents an integer of 1 or 2; and Y represents a halogen atom.
(13) The process according to item (11) or (12), wherein R1 is a nitro group.
(14) The process according to any one of items (11) to (13), wherein X in formula (I) is a group that bonds to the benzene ring via a nitrogen or sulfur atom.
(15) The process according to item (12), wherein the compound represented by formula (IIb) and synthesized by causing the compound represented by formula (III) to react with the compound represented by formula (IVb) is used, without being isolated, in the form of a reaction mixture.
(16) The process according to any one of items (11) to (15), wherein m in formula (I) is 1.
(17) The process according to any one of items (11) to (16), wherein p in formula (IIb) is 2.
(18) The process according to any one of items (11) to (17), wherein R3 and R4 in formula (IIb) each are an alkyl group.
(19) The process according to the item (12), wherein Y in formula (IVb) is a chlorine atom.
(Herein, the process for producing the compound represented by formula (I) as described in the above items (11) to (19) are collectively referred to as a second embodiment of the present invention.)

Herein, the present invention means to include both the first embodiment and the second embodiment, unless otherwise specified.

Hereinafter, the present invention is described in detail.

In the present invention, the compound represented by formula (II) can be represented by either formula (IIa) or formula (IIb). Therefore, unless otherwise specified, descriptions on the "formula (II)" are applied to both of formulas (IIa) and (IIb).

Further, in the present invention, the compound represented by formula (IV) can be represented by either formula (IVa) or formula (IVb). Therefore, unless otherwise specified, descriptions on the "formula (IV)" are applied to both of formulas (IVa) and (IVb).

The first embodiment of the present invention is a process for producing a compound represented by formula (I), by causing a compound represented by formula (IIa) to react with a nucleophilic agent. More specifically, the first embodiment is a process shown as the second step in the following reaction scheme A; and it is preferably a process of synthesizing a compound represented by formula (IIa) by reaction of a phenolic compound represented by formula (III), with a compound represented by formula (IVa), and then producing a compound represented by formula (I) therefrom. This process is preferably composed of first and second steps in the following reaction scheme A.

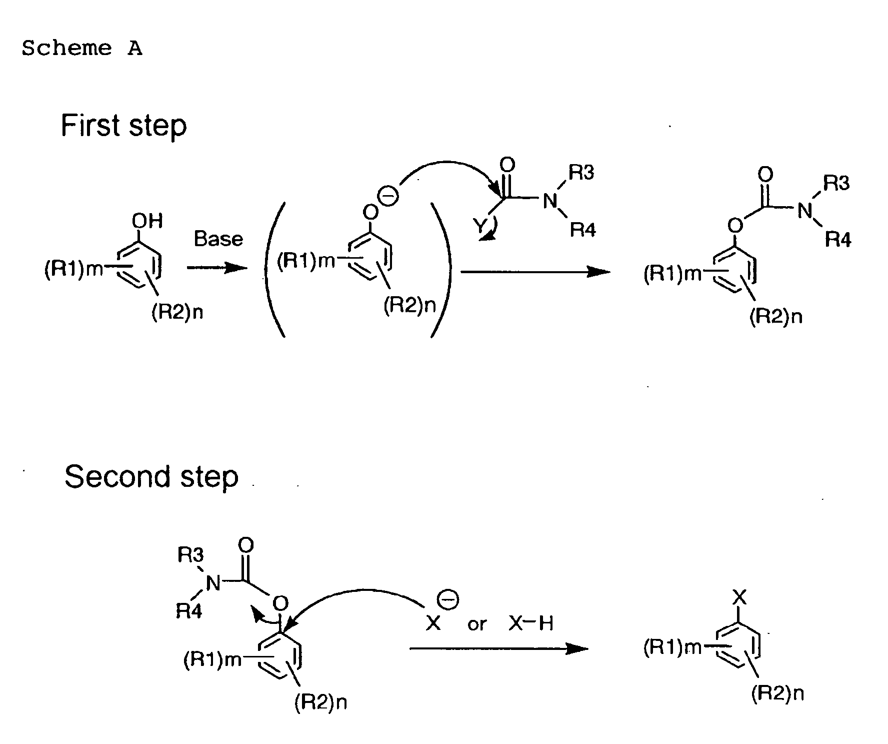

The second embodiment of the present invention is a process for producing a compound represented by formula (I), by causing a compound represented by formula (IIb) to react with a nucleophilic agent. More specifically, the second embodiment is a process shown as the second step in the following reaction scheme B; and it is preferably a process of synthesizing a compound represented by formula (IIb) by reaction of a phenolic compound represented by formula (III), with a compound represented by formula (IVb), and then producing a compound represented by formula (I) therefrom. This process is preferably composed of first and second steps in the following reaction scheme B.

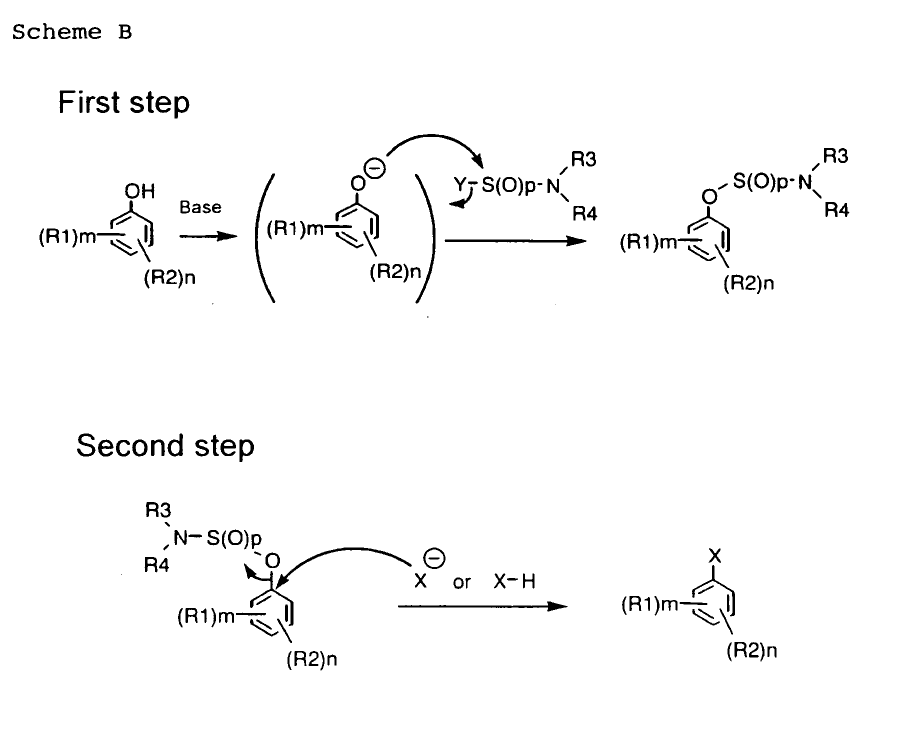

In the present specification, unless otherwise specified, the term "reaction scheme" refers to both Schemes A and B.

Further, unless otherwise specified, descriptions on the "first step" and the "second step" are applied to both of schemes A and B.

In the present specification, when the number of carbon atoms is described for a specific group, the number means the total of the carbon atoms contained in the group and its substituent(s), if the group has any substituent(s).

Next, the compounds represented by formula (I), formula (II), formula (III), or formula (IV), for use in the present invention, are explained in detail.

In formula (I), X represents a substituent. Examples of the substituent include a halogen atom, an alkyl group (including a cycloalkyl group and a bicycloalkyl group), an alkenyl group (including a cycloalkenyl group and a bicycloalkenyl group), an alkynyl group (including a cycloalkynyl group), an aryl group, a heterocyclic group, a cyano group, an alkoxy group, an aryloxy group, a heterocyclic oxy group, an acyloxy group, a carbamoyloxy group, an alkoxycarbonyloxy group, an aryloxycarbonyloxy group, an amino group (including an alkylamino group, an anilino group, a cyclic amino group), an acylamino group, an aminocarbonylamino group, an alkoxycarbonylamino group, an aryloxycarbonylamino group, a sulfamoylamino group, an alkyl- or aryl-sulfonylamino group, a mercapto group, an alkylthio group, an arylthio group, a heterocyclic thio group, a thiocyano group, an isothiocyano group, a sulfamoyl group, a sulfo group, an alkyl- or aryl-sulfinyl group, an alkyl- or aryl-sulfonyl group, an imido group, a phosphino group, a phosphinyl group, a phosphinyloxy group, a phosphinylamino group. X is preferably a substituent other than a hydroxyl group; since, when X is a hydroxyl group, the target compound represented by formula (I) returns to the compound represented by formula (III), which is the raw material.

The above-mentioned substituent may be further substituted with a substituent, examples of which include the above-mentioned groups.

X is preferably a group having 30 or less carbon atoms in sum total. Preferred number of carbon atoms in X is: for example, 1 to 30 carbon atoms when X is an alkyl group, an alkoxy group, an acyloxy group, a carbamoyloxy group, an acylamino group, an aminocarbonylamino group, an alkyl sulfonylamino group, an alkylsulfinyl group, an alkylsulfonyl group, or an alkylthio group; 2 to 30 carbon atoms when X is an alkenyl group, an alkynyl group, an alkoxycarbonyloxy group, or an alkoxycarbonylamino group; 3 to 30 carbon atoms when X is a cycloalkyl group or a cycloalkenyl group; 6 to 30 carbon atoms when X is a cycloalkynyl group, a bicycloalkenyl group, an aryl group, an aryloxy group, an arylsulfonylamino group, an arylthio group, an arylsulfinyl group, or an arylsulfonyl group; 0 to 30 carbon atoms when X is a heterocyclic group, a heterocyclic oxy group, an amino group, a sulfamoylamino group, a heterocyclic thio group, a sulfamoyl group, a phosphino group, a phosphinyl group, a phosphinyloxy group, or a phosphinylamino group; 7 to 30 carbon atoms when X is an aryloxycarbonyloxy group, or an aryloxycarbonylamino group; and 4 to 30 carbon atoms when X is an imido group.

X is preferably a group that bonds to the benzene ring via a nitrogen, sulfur, oxygen, or carbon atom, more preferably a group that bonds to the benzene ring via a nitrogen or sulfur atom, even more preferably a group that bonds to the benzene ring via a sulfur atom, and most preferably an alkylthio or arylthio group.

Examples of the nucleophilic agent caused to react with the compound represented by formula (II) include X anions, and compounds each having a hydrogen atom or metallic atom bonded to X. In the above, X is a structure having the same meaning as X in formula (I).

Examples of the nucleophilic agent include amines (such as ammonia, diethylamine, dodecylamine, and pyrrolidine), anilines (such as aniline, and N-methylaniline), alcohols (such as methanol, ethanol, i-propanol, and n-dodecanol), phenols (such as phenol, and p-chlorophenol), thiols (such as methylmercaptan, i-propylmercaptan, and 2-ethylhexylmercaptan), thiophenols (such as thiophenol, and p-dodecylthiophenol), imides (such as succinimide); active methylene compounds (such as dimethyl malonate, and ethyl acetoacetate), and salts thereof; alkyl- or aryl-Grignard reagents (such as a methyl Grignard reagent, and a phenyl Grignard reagent); alkyl- or aryl-lithium compounds (such as n-butyllithium); cyanides; sulfides; azides; halides; cyanates; thiocyanates; and sulfites.

Of these nucleophilic agents, preferred are nucleophilic agents wherein the atom that nucleophilically attacks one of the carbons in the benzene ring to bond to the carbon atom is nitrogen, sulfur, oxygen, or carbon. More preferred are nucleophilic agents wherein the attacking atom is nitrogen or sulfur, and even more preferred are nucleophilic agents wherein the attacking atom is sulfur. Of the nucleophilic agents, most preferred are alkyl- or aryl-thiols, or anions or salts thereof.

The alkylthiols, or anions or salts thereof are preferably those having 1 to 30 carbon atoms, and the arylthiols, or anions or salts thereof are preferably those having 6 to 30 carbon atoms. Examples of these include methylthiol, butylthiol, 2-ethylhexylthiol, octylthiol, phenylthiol, or an anion or salt thereof. Among these, the alkylthiols having 1 to 30 carbon atoms, or anions or salts thereof are more preferably used; and, of these, 2-ethylhexylthiol, or an anion or salt thereof is most preferred.

In formulae (I), (II), and (III), R1 represents an electron-withdrawing substituent having a Hammett substituent constant σₚ of more than 0. The Hammett substituent constant σₚ is preferably from 0 to 1.5 (inclusive).

Herein, Hammett substituent constant σₚ is described in detail in such books as "Hammett Soku - Kozo to Hannousei -," written by Naoki Inamoto (Maruzen); "Shinjikken Kagaku-koza 14/Yukikagoubutsu no Gosei to Hanno V," page 2605 (edited by Nihonkagakukai, Maruzen); "Riron Yukikagaku Kaisetsu," written by Tadao Nakaya, page 217 (Tokyo Kagakudojin); and "Chemical Review" (Vol. 91), pages 165 to 195 (1991).

Examples of R1 include a halogen atom, a formyl group, an acyl group, an alkoxycarbonyl group, an aryloxycarbonyl group, an alkyl- or aryl-sulfonyl group, an alkyl- or aryl-sulfinyl group, a carbamoyl group, a sulfamoyl group, a cyano group, a nitro group, a halogen-substituted alkyl or alkoxy group (such as trifluoromethyl and trifluromethoxy), an acyloxy group, and an acylamide group.

R1 may have a substituent, examples of which are the same as described above as examples of the substituent of X. R1 is preferably an electron-withdrawing substituent having a Hammett σₚ constant of more than 0.4. Examples of the electron-withdrawing group having a Hammett σₚ constant of more than 0.4 include an alkyloxycarbonylthio group, an alkoxycarbonyl group, an aryloxycarbonyl group, an alkylsulfinyl group, an arylsulfinyl group, an alkylsulfonyl group, an arylsulfonyl group, an acyl group, a perfluoroalkyl group, perfluoroalkylthio group, a sulfamoyl group, a cyano group, and a nitro group. R1 is more preferably an electron-withdrawing substituent having a Hammett σₚ constant of more than 0.6. Examples of the electron-withdrawing group having a Hammett σₚ constant of more than 0.6 include a cyano group, an alkylsulfonyl group, an arylsulfonyl group, and a nitro group. R1 is most preferably a nitro group.

In formulae (I), (II), and (III), m represents an integer of 1 to 5, preferably an integer of 1 to 3, more preferably an integer of 1 or 2, and most preferably 1. When m is 1, R1 is most preferably substituted on the ortho or para position to the position of X.

In formulae (I), (II), and (III), R2 represents a substituent. Examples of this substituent are the same as described above as examples of the substituent of X.

R2 is preferably an alkyl group, an alkenyl group, an aryl group, a heterocyclic group, an alkoxy group, an aryloxy group, an alkylthio group, an arylthio group, a heterocyclic thio group, a halogen atom, an amino group, an acylamino group, a sulfonamido group, an imido group, a cyano group, a nitro group, an acyl group, an alkoxycarbonyl group, an aryloxycarbonyl group, an alkyl- or aryl-sulfonyl group, an alkyl- or aryl-sulfinyl group, a carbamoyl group, and a sulfamoyl group.

In formulae (I), (II), and (III), n is an integer of 0 to 4, preferably an integer of 0 to 3, and more preferably an integer of 0 to 2.

The total of m and n (i.e., m + n) is an integer of 1 to 5.

In formulae (I), (II), and (III), plural R1s, plural R2s, or R1 and R2 may bond to each other to form a ring. This ring may be aromatic, non-aromatic, alicyclic, or heterocyclic.

In formula (II) and formula (IV), R3 and R4 each independently represent an alkyl group, an aryl group, or a heterocyclic group.

The alkyl group is preferably a substituted or unsubstituted, straight- or branched-chain alkyl group (including cycloalkyl) having 1 to 30 carbon atoms. Examples of the alkyl group include a methyl group, an ethyl group, a propyl group, an i-propyl group, a t-butyl group, a dodecyl group, a 2-ehtylhexyl group, and a cyclohexyl group.

The aryl group is preferably a substituted or unsubstituted, single or condensed ring, aryl group having 6 to 30 carbon atoms. Examples of the aryl group include a phenyl group, a p-tolyl group, a naphthyl group, a m-chlorophenyl group, and an o-hexadecanoylaminophenyl group.

The heterocyclic group is preferably a 5- or 6-membered ring, and may be condensed to another ring. The heterocyclic group may be an aromatic heterocyclic group or a non-aromatic heterocyclic group. Examples of the heterocycle in the heterocyclic group include pyridine, pyrazine, pyrimidine, pyridazine, triazine, quinoline, isoquinoline, quinazoline, cinnoline, phthalazine, quinoxaline, pyrrole, indole, furan, benzofuran, thiophene, benzothiophene, pyrazole, imidazole, benzimidazole, triazole, oxazole, benzoxazole, thiazole, benzothiazole, isothiazole, benzisothiazole, thiadiazole, isoxazole, benzisoxazole, pyrrolidine, piperidine, piperazine, imidazolidine, and thiazoline.

In formulae (IIa) and (IVa), R3 and R4 each are preferably an alkyl group, more preferably an alkyl group having 2 or more carbon atoms, even more preferably an ethyl or i-propyl group, and most preferably an i-propyl group.

In formulae (IIb) and (IVb), R3 and R4 each are preferably an alkyl group, more preferably a methyl or ethyl group, and most preferably a methyl group.

In formulae (II) and (IV), R3 and R4 may bond to each other to form a ring. This ring may be aromatic, non-aromatic, alicyclic, or heterocyclic, and is preferably non-aromatic. Examples of the ring formed by R3 and R4 bonded together include a pyrrolidine ring, a piperidine ring, a piperazine ring, a morpholine ring, a pyrrole ring, an imidazole ring, a pyrazole ring, an indole ring, and an indoline ring. Among these, a pyrrolidine ring, a piperidine ring, a piperazine ring, and a morpholine ring are more preferred.

In formulae (II), (IIb), (IV), and (IVb), p represents 1 or 2, and preferably p is 2.

In formula (IV), Y represents a halogen atom. Examples of the halogen atom include fluorine, chlorine, bromine, and iodine atoms. Y is preferably a chlorine atom.

Specific examples of the compound represented by formula (I) according to the present invention are shown below, but the present invention is not limited to these specific examples. In the compounds shown below, Me represents a methyl group and Et represents an ethyl group (the same rule is correspondingly applied to the whole of the description).

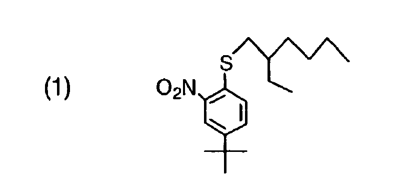

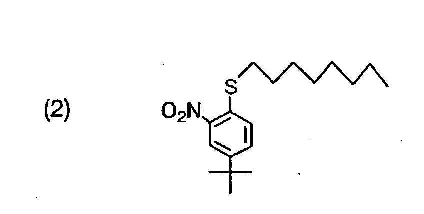

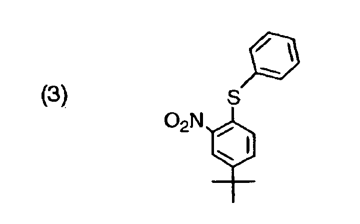

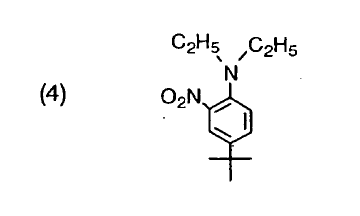

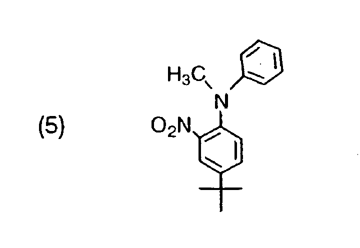

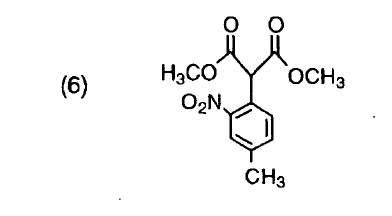

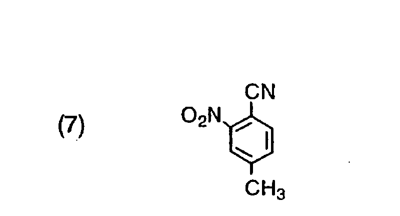

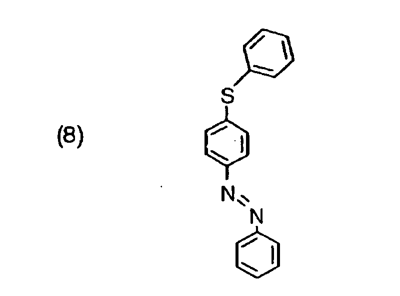

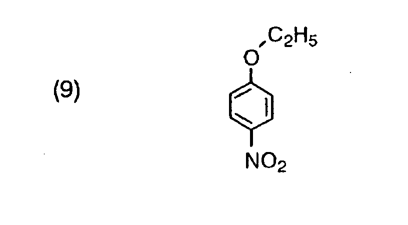

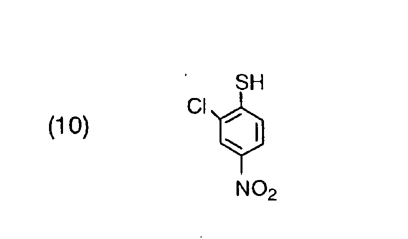

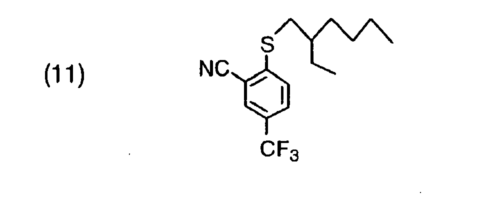

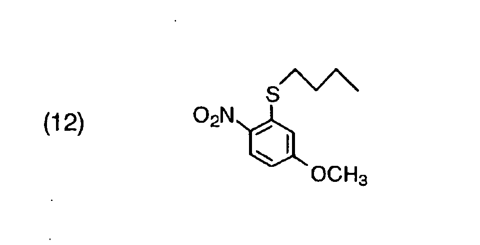

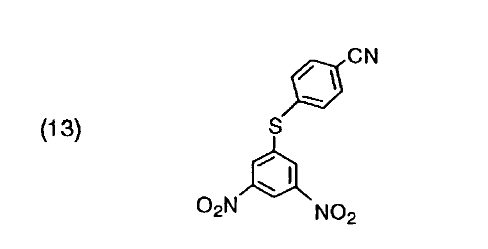

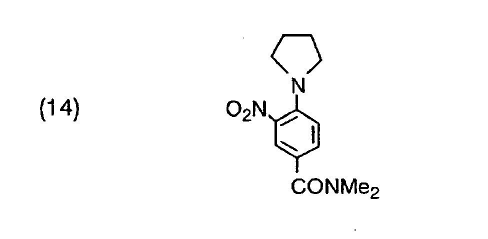

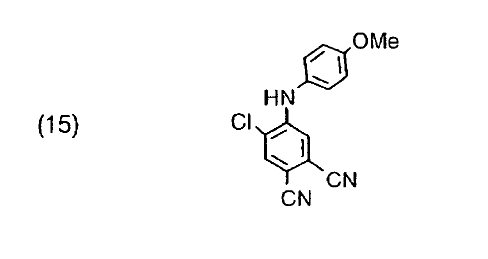

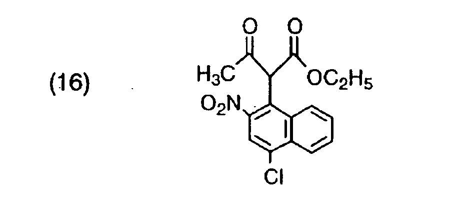

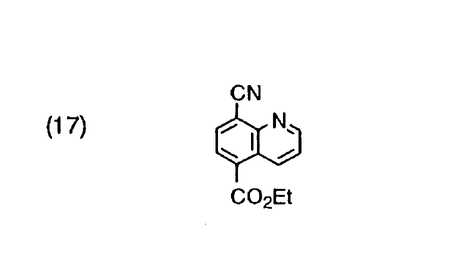

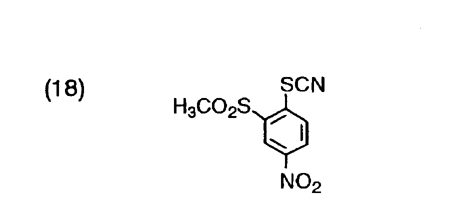

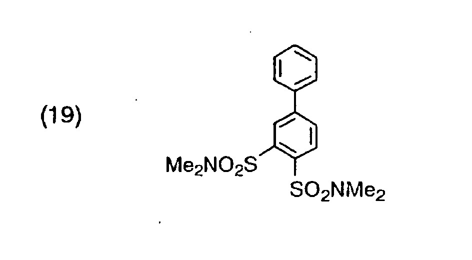

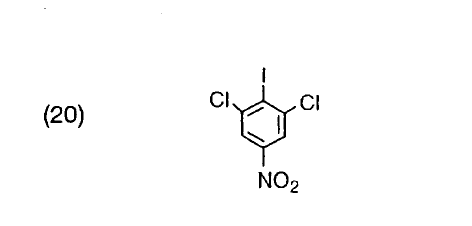

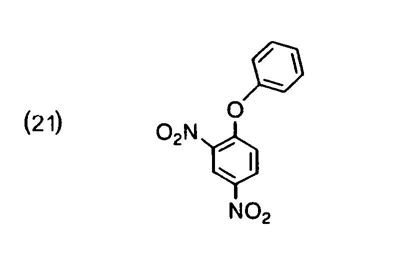

Specific examples of the compound represented by formula (IIa) according to the present invention are shown below, but the present invention is not limited to these specific examples.

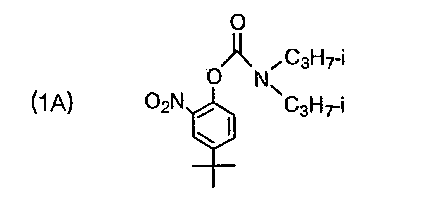

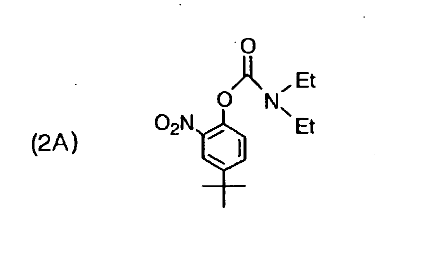

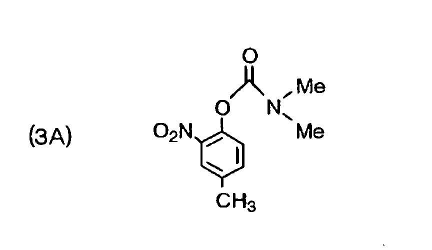

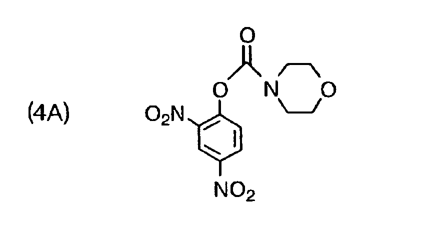

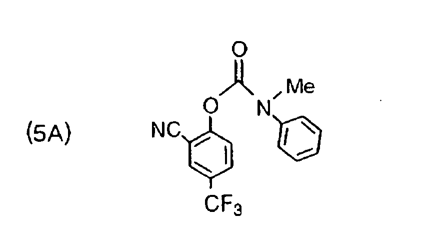

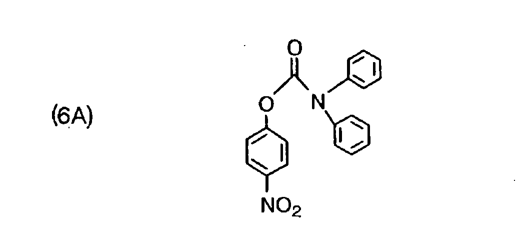

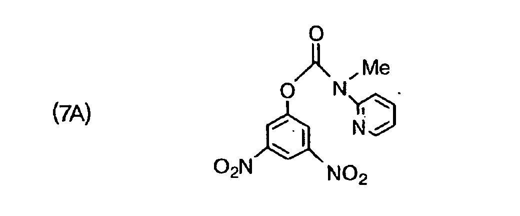

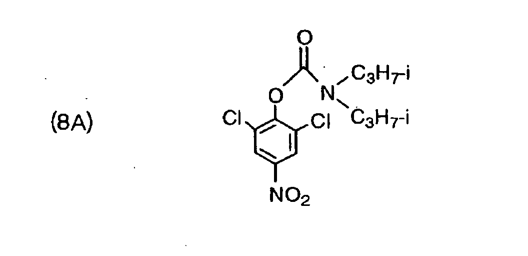

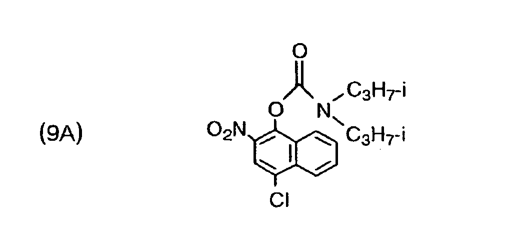

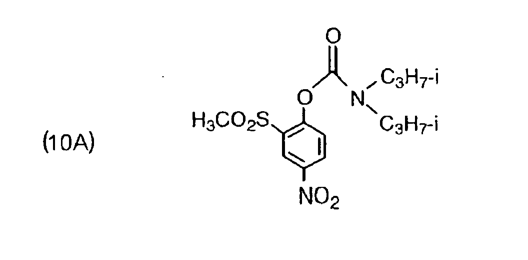

Specific examples of the compound represented by formula (IIb) according to the present invention are shown below, but the present invention is not limited to these specific examples.

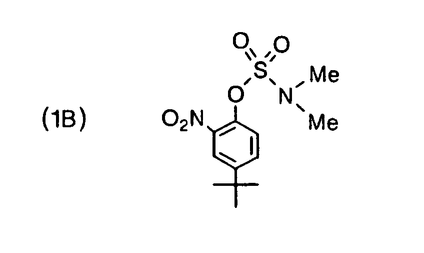

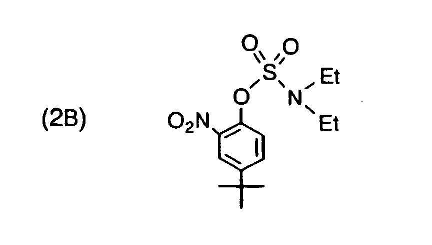

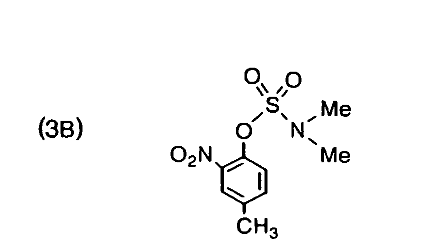

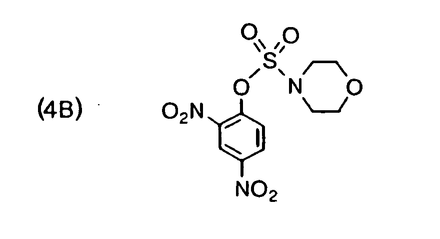

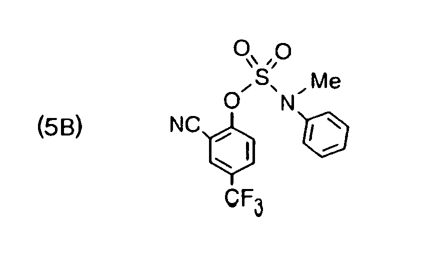

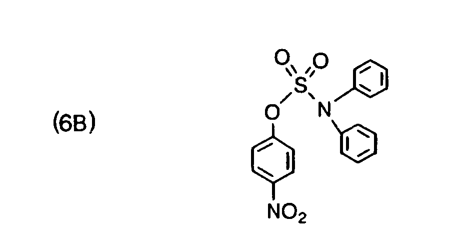

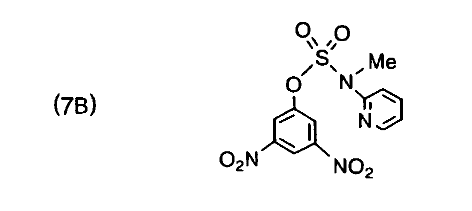

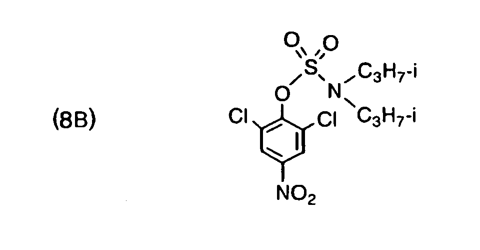

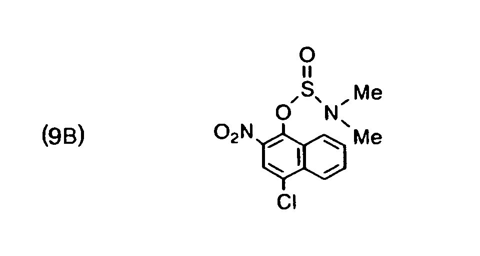

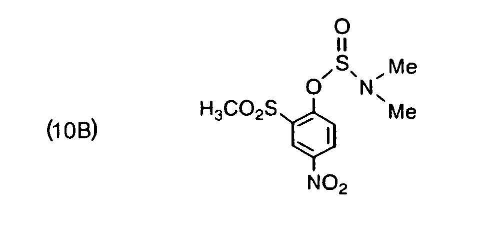

The phenolic compound represented by formula (III) can be synthesized by known various synthesis methods.

The compound represented by formula (IVa) is known, and is commercially available, or can easily be produced by a known method.

The compound represented by formula (IVb) can be synthesized by known various synthesis methods. In the case that Y is, for example, a chlorine atom, a method of synthesizing the compound from sulfuryl chloride and dimethylamine by a method described in JP-A-3-127769 ("JP-A" means unexamined published Japanese patent application) is inexpensive and preferable.

Each of the first step and the second step in the reaction scheme A or B may be performed or both of the steps may be continuously performed without using any solvent or in the state that the reactants are dissolved or dispersed in an appropriate solvent. Examples of the solvent which can be used in the reaction according to the present invention include water, alcohol-based solvents (such as methanol and isopropanol), chlorine-containing solvents (such as dichloromethylene), aromatic solvents (such as benzene, chlorobenzene, and toluene), amide-based solvents (such as N,N-dimethylformamide, N,N-dimethylacetoamide, and N-methylpyrrolidone), nitrile-based solvents (such as acetonitrile, and propionitrile), ether-based solvents (such as tetrahydrofuran, ethylene glycol diethyl ether), sulfone-based solvents (such as dimethylsulfone, and sulfolane), phosphoric amide-based solvents (such as hexamethylphosphoric triamide), and hydrocarbon-based solvents (such as cyclohexane, and n-hexane).

These solvents may be used alone or in the form of a mixture of two or more thereof. It is particularly preferable that the reaction is performed in a two-phase system wherein water and an organic solvent immiscible with water are used together. When the first and second steps are performed without interruption, a solvent different from the solvent used in the first step may be used in the second step. The amount of the solvent(s) is usually from 0.1 to 1,000 parts by mass, preferably from 0.5 to 100 parts by mass, and more preferably from 1 to 50 parts by mass per part by mass of the compound represented by formula (III) in the first step and per part by mass of the compound represented by formula (II) in the second step, respectively.

In the process of the present invention, a base is preferably used in the first or second step, more preferably in both of the first and second steps. Examples of the base which can be used in the present invention include hydroxide compounds (such as sodium hydroxide, potassium hydroxide, and tetrabutylammonium hydroxide), carbonate compounds (such as sodium carbonate, potassium carbonate, and tetrabutylammonium carbonate), amines (such as ammonia, and triethylamine), anilines (such as diethylaniline), aromatic heterocyclic compounds (such as pyridine, and imidazole), guanidines (such as tetramethylguanidine), bicarbonate compounds (such as sodium bicarbonate), nitrogen-containing heterocyclic compounds (such as 1,8-diazabicyclo[5,4,0]-7-undecene), acetic acid compounds (such as sodium acetate, and potassium acetate), and metal salts of organic compounds (such as sodium methoxide).

These bases may be used alone or in the form of a mixture of two or more thereof. The amount of the base(s) is usually from 0.1 to 100 moles, preferably from 0.5 to 10 moles, and more preferably from 1 to 5 moles per mole of the compound represented by formula (III) in the first step and per mole of the compound represented by formula (II) in the second step, respectively.

In the process of the present invention, it is also preferable to use a phase-transfer catalyst in the first or second step or in both of the first and second steps. Examples of the phase-transfer catalyst which can be used in the present invention include ammonium salts (such as tetrabutylammonium bromide, benzyltrimethylammonium chloride, and tetraethylammonium iodide), and phosphinium salts (such as tetrabutylphosphinium bromide). The ammonium salts are preferably used.

The amount of the phase-transfer catalyst is preferably from 0.001 to 10 moles, more preferably from 0.01 to 1 mole, even more preferably from 0.05 to 0.5 mole, and particularly preferably from 0.02 to 0.5 mole per mole of the compound represented by formula (I).

It is particularly preferable to use the phase-transfer catalyst at least in the second step, and this is particularly preferable when X in formula (I) is an alkylthio or arylthio group. When the phase-transfer catalyst is used, the reaction solvents are preferably water and an aromatic solvent (such as toluene).

The ratio of water to an aromatic solvent is preferably in the range of 1:0.1 to 1:20, and more preferably 1:1 to 1:20, in terms of volume ratio.

In the second step, the amount of the nucleophilic agent is usually from 0.01 to 100 moles, preferably from 0.1 to 50 moles, more preferably from 0.2 to 20 moles, and even more preferably from 0.5 to 10 moles per mole of the compound represented by formula (II).

In the second step, the compound represented by formula (II) prepared in the first step may be used after being isolated. However, it is preferable to use the solution containing the compound represented by formula (II) as it is, without isolating the compound represented by formula (II).

In the second step, a nucleophilic agent or a solution thereof may be added to the compound represented by formula (II) or solution thereof prepared in the first step. Alternatively, the compound represented by formula (II) or the solution thereof may be added to a nucleophilic agent or a solution thereof.

The reaction temperature for the present reaction is not particularly limited in each of the first and second steps. The reaction can be carried out from -50 to 150 °C, preferably from -10 to 100 °C, and more preferably from 0 to 80 °C.

The compound represented by formula (I) according to the present invention is useful as a synthesis intermediate of photographically-useful compounds to be used in silver halide photographic light-sensitive materials, or as a synthesis intermediate of medical supplies, agricultural chemicals, or the like. The compound represented by formula (I) is preferably used as a synthesis intermediate of a photographically-useful compound to be used in silver halide photographic light-sensitive materials, such as a dye-forming coupler, a dye, and a ballast group in a hydrophobic additives. Among these, the compound is preferably used to form a dye-forming coupler and a dye. Specifically, the compound represented by formula (I) is preferably applied to yellow dye-forming couplers described in JP-A-2003-173007, JP-A-2004-118082, JP-A-2004-118109, and JP-A-2004-139062; cyan dye-forming couplers described in JP-A-11-282138; and magenta dye-forming couplers described in JP-A-8-122984. For example, dye-forming couplers can be obtained by reducing a nitro group in the compound represented by formula (I) to an amino group, and then further reacting the amino group. Synthesis of these compounds can be carried out according to the methods described in the above-mentioned publications. Especially, yellow dye-forming couplers described in JP-A-2003-173007, JP-A-2004-118082, JP-A-2004-118109, and JP-A-2004-139062 can be synthesized, according to the synthesis of Coupler (2) from Compound (A-4) described in Paragraph 0072 in JP-A-2004-139062.

The present invention overcomes problems in the prior art, and the present invention can produce an aromatic compound derivative that is inexpensive and widely usable, at high yield, through a simple process. In particular, the present invention makes it possible to synthesize an aromatic compound derivative, with high selectivity and high yield, easily and inexpensively through a short process, using a compound that can be derived from a phenolic compound.

According to the present invention, it is possible to synthesize a derivative of an aromatic compound having a substituent, which compound is useful as a synthesis intermediate of photographically-useful compounds for use in a silver halide photographic photosensitive material, or medical supplies, agricultural chemicals, or others.

Hereinafter, specific examples of the synthesis of the present invention are described. However, the present invention is not limited thereto.

### EXAMPLES

### Example 1-1: Synthesis of Exemplified compound (1)

The compound was synthesized in accordance with following reaction scheme.

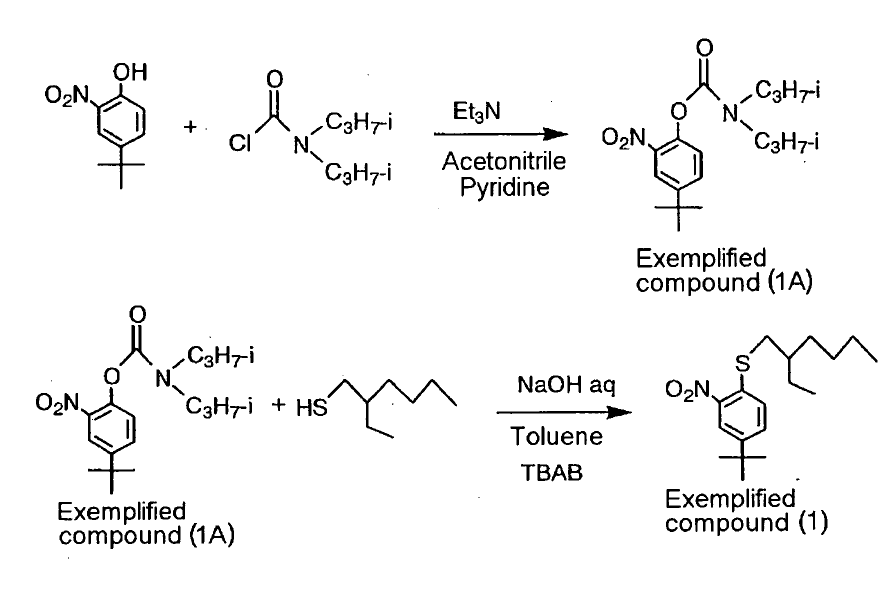

Into 50 mL of acetonitrile were dissolved 5.80 g (29.7 mmol) of 4-t-butyl-2-nitrophenol and 5.00 g (30.6 mmol) of N,N-diisopropylcarbamoyl chloride, and then, to the resultant solution, were added 4.8 mL of triethylamine and 1.0 mL of pyridine. The resultant solution was stirred at 60 °C for 5 hours. To the reaction solution were added ethyl acetate and water, to extract the reaction product. The organic phase was washed with each of 0.1N potassium carbonate aqueous solution, diluted hydrochloric acid, and saturated brine, followed by drying over anhydrous magnesium sulfate. The result was filtered and then the solvent was distilled off under reduced pressure. The remaining product was purified by silica gel chromatography, using a mixed solvent of ethyl acetate and hexane as an eluent, to yield 8.82 g (27.4 mmol) of Exemplified compound (1A), as a light yellow crystal. Yield: 92.1%. ¹H NMR (400MHz, CDCl₃) δ1.31-1.34 (12H, m), 1.35 (9H, s), 3.85-3.89 (1H, m), 4.17-4.21 (1H, m), 7.18 (1H, d, J=8.4Hz), 7.62 (1H, dd, J=8.4, 2.4Hz), 8.04 (1H, d, J=2.4Hz).

Into 10 ml of toluene were dissolved 3.22 g (0.010 mol) of Exemplified compound (1A), 1.76 g (0.012 mol) of 2-ethylhexylmercaptan, and 0.32 g (0.0010 mol) of tetrabutylammonium bromide (TBAB), and then to that was added 6 mL of 2M sodium hydroxide aqueous solution. The resultant solution was stirred at 80 °C for 11 hours. To the reaction solution were added ethyl acetate and water, to extract the reaction product. The organic phase was washed with diluted hydrochloric acid and saturated brine, followed by drying over anhydrous magnesium sulfate. The result was filtered, and then the solvent was distilled off under reduced pressure. The remaining product was purified by silica gel chromatography, using a mixed solvent of ethyl acetate and hexane as an eluent, to yield 2.75 g (0.00850 mol) of Exemplified compound (1), as a yellow oil substance. Yield: 85.0%. ¹H NMR (400MHz, CDCl₃) δ0.88-1.56 (14H, m), 1.35 (9H, s), 1.65-1.71 (1H, m), 2.89 (2H, d, J=6.3Hz), 7.36 (1H, d, J=8.6Hz), 7.57 (1H, dd, J=8.6, 2.2Hz), 8.18 (1H, d, J=2.2Hz).

### Example 1-2: Synthesis of Exemplified compound (1)

The compound was synthesized in accordance with following reaction scheme.

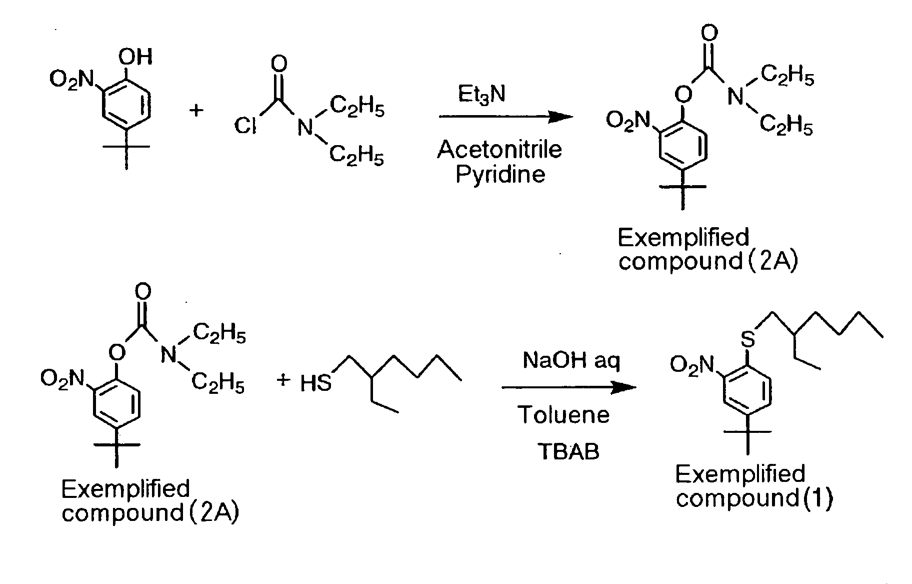

Into 50 mL of acetonitrile were dissolved 9.75 g (0.050 mol) of 4-t-butyl-2-nitrophenol and 8.13 g (0.060 mol) of N,N-diethylcarbamoyl chloride, and then, to the resultant solution, were added 8.4 mL of triethylamine and 1.0 mL of pyridine. The resultant solution was stirred at 50 °C for 6 hours. To the reaction solution were added ethyl acetate and water, to extract the reaction product. The organic phase was washed with each of a 0.1N potassium carbonate aqueous solution, diluted hydrochloric acid, and saturated brine, followed by drying over anhydrous magnesium sulfate. The result was filtered and then the solvent was distilled off under reduced pressure. The remaining product was purified by silica gel chromatography, using a mixed solvent of ethyl acetate and hexane as an eluent, to yield 14.3 g (0.043 mol) of Exemplified compound (2A), as a light yellow oil substance. Yield: 86.6%. ¹H NMR (400MHz, CDCl₃) δ1.22 (3H, t, J=7.2Hz), 1.28 (3H, t, J=7.2Hz), 1.35 (9H, s), 3.38 (2H, q, J=7.2Hz), 3.48 (2H, q, J=7.2Hz), 7.21 (1H, d, J=8.2Hz), 7.62 (1H, dd, J=8.2, 2.4Hz), 8.04 (1H, d, J=2.4Hz).

Into 6 ml of toluene were dissolved 2.95 g (0.010 mol) of Exemplified compound (2A), 1.72 g (0.012 mol) of 2-ethylhexylmercaptan, and 0.32 g (0.0010 mol) of tetrabutylammonium bromide, and then, to that was added 6 mL of 2M sodium hydroxide aqueous solution. The resultant solution was stirred at 80 °C for 10 hours. To the reaction solution were added ethyl acetate and water, to extract the reaction product. The organic phase was washed with diluted hydrochloric acid and saturated brine, followed by drying over anhydrous magnesium sulfate. The result was filtered, and then the solvent was distilled off under reduced pressure. The remaining product was purified by silica gel chromatography, using a mixed solvent of ethyl acetate and hexane as an eluent, to yield 2.60 g (0.00804 mol) of Exemplified compound (1), as a yellow oil substance. Yield: 80.4%. ¹H NMR (400MHz, CDCl₃) δ0.88-1.56 (14H, m), 1.35 (9H, s), 1.65-1.71 (1H, m), 2.89 (2H, d, J=6.3Hz), 7.36 (1H, d, J=8.6Hz), 7.57 (1H, dd, J=8.6, 2.2Hz), 8.18 (1H, d, J=2.2Hz).

### Example 2-1: Synthesis of Exemplified compound (1)

The compound was synthesized in accordance with following reaction scheme.

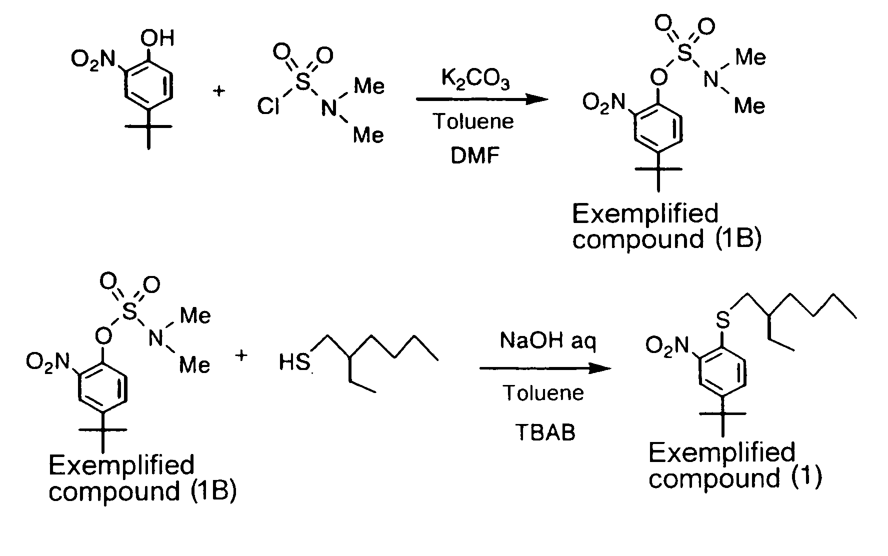

Into 90 mL of toluene and 18 mL of N,N-dimethylformamide (DMF) were dissolved 29.3 g (0.150 mol) of 4-t-butyl-2-nitrophenol, and 22.6 g (0.157 mol) of N,N-dimethylsulfamoyl chloride, and then, to that was added 24.8 g (0.180 mol) of potassium carbonate. The resultant solution was then stirred at 70 °C for 4 hours. To the reaction solution were added ethyl acetate and water, to extract the reaction product. The organic phase was washed with 0.1N potassium carbonate aqueous solution, diluted hydrochloric acid, and saturated brine, followed by drying over anhydrous magnesium sulfate. The result was filtered, and then the solvent was distilled off under reduced pressure. The remaining product was crystallized from a mixed solvent of ethyl acetate and hexane, to yield 36.3 g (0.120 mol) of Exemplified compound (1B), as a light yellow crystal. Yield: 80.0%.

Into 15 ml of toluene were dissolved 3.02 g (0.010 mol) of Exemplified compound (1B), 1.75 g (0.012 mol) of 2-ethylhexylmercaptan, and 0.28 g (0.0010 mol) of tetrabutylammonium bromide (TBAB), and then, to that was added 5 mL of 25% sodium hydroxide aqueous solution. The resultant solution was stirred at 60 °C for 1 hour. To the reaction solution were added ethyl acetate and water, to extract the reaction product. The organic phase was washed with diluted hydrochloric acid and saturated brine, followed by drying over anhydrous magnesium sulfate. The result was filtered, and then the solvent was distilled off under reduced pressure. The remaining product was purified by silica gel chromatography, using a mixed solvent of ethyl acetate and hexane as an eluent, to yield 3.05 g (0.00943 mol) of Exemplified compound (1), as a yellow oil substance. Yield: 94.3%. ¹H-NMR (400MHz, CDCl₃) δ0.88-1.56 (14H, m), 1.35 (9H, s), 1.65-1.71 (1H, m), 2.89 (2H, d), 7.36 (1H, d), 7.57 (1H, dd), 8.18 (1H, d).

### Example 2-2: Synthesis of Exemplified compound (4)

The compound was synthesized in accordance with following reaction scheme.

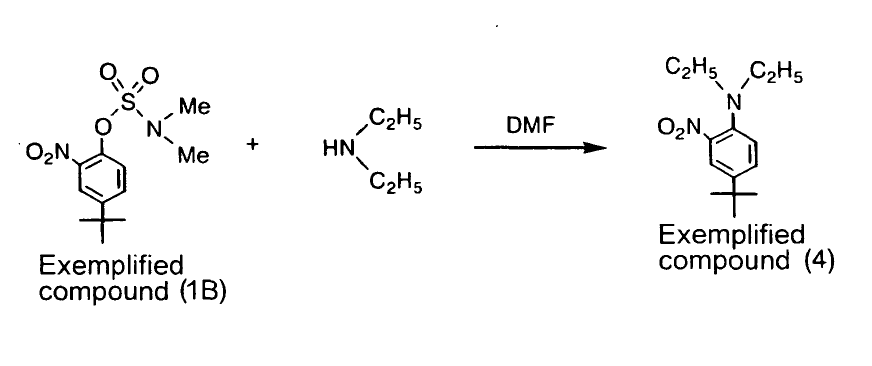

Into 15 mL of DMF were dissolved 3.02 g (0.010 mol) of Exemplified compound (1B) and 2.19 g (0.030 mol) of diethyamine. The resultant solution was stirred at 80 °C for 8 hours. To the reaction solution were added ethyl acetate and water, to extract the reaction product. The organic phase was washed with diluted hydrochloric acid, and saturated brine, followed by drying over anhydrous magnesium sulfate. The result was filtered and then the solvent was distilled off under reduced pressure. The remaining product was purified by silica gel chromatography, using a mixed solvent of ethyl acetate and hexane as an eluent, to yield 2.26 g (0.0903 mol) of Exemplified compound (4), as an orange yellow oil substance. Yield: 90.3%. ¹H-NMR (400MHz, CDCl₃) δ1.07 (6H, t), 1.33 (9H, t), 3.12(4H, q), 7.11 (1H, d), 7.44 (1H, dd), 7.62 (1H, d).

### Example 2-3: Synthesis of Exemplified compound (1)

The compound was synthesized in accordance with following reaction scheme.

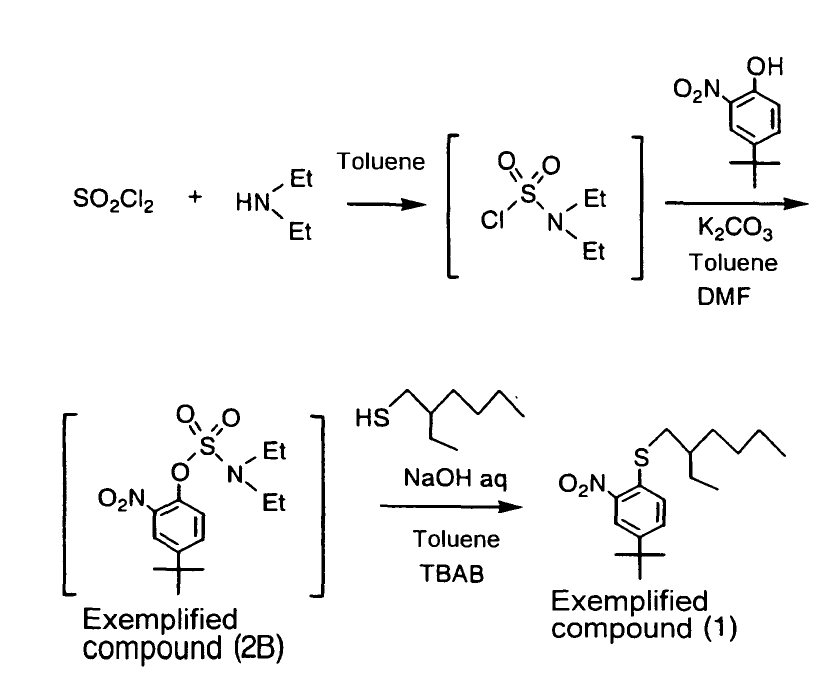

To a solution of 16.2 g (0.120 mol) of sulfuryl chloride in 50 mL of toluene, was added dropwise, 17.5 g (0.240 mol) of diethylamine in 25 mL of toluene, over 1 hour, with ice cooling. The solution was stirred for 4 hours, with ice cooling, and then cold water was added to it, to perform extraction. Further, the resultant solution was washed with cold water, twice, to yield a solution of N,N-diethylsulfamoyl chloride in toluene.

The resultant solution of N,N-diethylsulfamoyl chloride in toluene was added to a solution containing 19.5 g (0.100 mol) of 4-t-butyl-2-nitrophenol and 16.6 g (0.120 mol) of potassium carbonate in 15 ml of N,N-dimethylformamide (DMF). The resultant solution was stirred at 70 °C for 4 hours. Ethyl acetate and water were added to the reaction solution, to extract the reaction product. The organic phase was washed with 0.1N potassium carbonate aqueous solution, diluted hydrochloric acid, and saturated brine, to yield a reaction solution of Exemplified compound (2B).

To the resultant reaction solution of Exemplified compound (2B) were added 17.5 g (0.120 mol) of 2-ethylhexylmercaptan and 2.78 g (0.010 mol) of tetrabutylammonium bromide, and to that was added 50 mL of 25% sodium hydroxide aqueous solution. The resultant solution was then stirred at 60 °C for 2 hours. To the reaction solution were added ethyl acetate and water, to extract the reaction product. The organic phase was washed with diluted hydrochloric acid and saturated brine, followed by drying over anhydrous magnesium sulfate. The result was filtered and then the solvent was distilled off under reduced pressure. The remaining product was purified by silica gel chromatography, using a mixed solvent of ethyl acetate and hexane as an eluent, to yield 29.2 g (0.0903 mol) of Exemplified compound (1), as a yellow oil substance. Yield: 90.3%. In the present example, nuclear magnetic resonance analysis (¹H-NMR, 400 MHz, CDCl₃) was performed, in the same manner as in Examples 2-1 and 2-2. As a result, the very same spectrum as in Example 2-1 was obtained as to the number of signals, the positions thereof, and splitting. Accordingly, the above-mentioned yellow oil substance was identified as Exemplified compound (1).

### Comparative Example 1: Synthesis of Exemplified compound (1)

The compound was synthesized via a p-toluenesulfonyl product, as illustrated in the following reaction scheme.

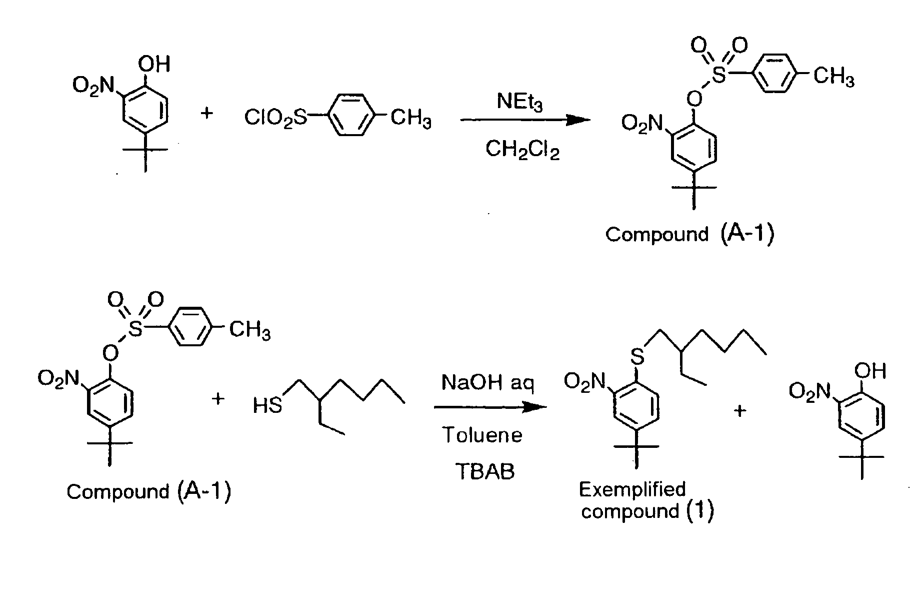

Into 100 mL of methylene chloride were dissolved 19.5 g (0.100 mol) of 4-t-butyl-2-nitrophenol and 16.7 mL (0.120 mol) of triethylamine, and then to that was added 21.0 g (0.110 mol) of p-toluenesulfonyl chloride, with ice cooling. The resultant solution was then stirred at room temperature for 4 hours. To the reaction solution were added ethyl acetate and water, to extract the reaction product. The organic phase was washed with 0.1N potassium carbonate aqueous solution, diluted hydrochloric acid, and saturated brine, followed by drying over anhydrous magnesium sulfate. The result was filtered, and then the solvent was distilled off under reduced pressure. The remaining product was crystallized from a mixed solvent of ethyl acetate and hexane, to yield 30.9 g (0.0884 mol) of Compound (A-1), as a light yellow crystal. Yield: 88.4%.

Into 15 ml of toluene were dissolved 3.49 g (0.010 mol) of Compound (A-1), 1.75 g (0.012 mol) of 2-ethylhexylmercaptan, and 0.28 g (0.0010 mol) of tetrabutylammonium bromide, and then to that was added 5 mL of 25% sodium hydroxide aqueous solution. The resultant solution was stirred at 60 °C for 1 hour. To the reaction solution were added ethyl acetate and water, to extract the reaction product. The organic phase was washed with diluted hydrochloric acid and saturated brine, followed by drying over anhydrous magnesium sulfate. The result was filtered, and then the solvent was distilled off under reduced pressure. The remaining product was purified by silica gel chromatography, using a mixed solvent of ethyl acetate and hexane as an eluent, to yield 1.46 g (0.00452 mol) of Exemplified compound (1) (Yield: 45.2%) and 0.94 g (0.00482 mol) of 4-t-butyl-2-nitrophenol. As described above, the selectivity of the reaction product was very low when the product was synthesized via the p-toluenesulfonyl product.

Having described our invention as related to the present embodiments, it is our intention that the invention not be limited by any of the details of the description, unless otherwise specified, but rather be construed broadly within its spirit and scope as set out in the accompanying claims.

## Claims

1. A process for producing a compound represented by the following formula (I), which comprises causing a compound represented by the following formula (II) to react with a nucleophilic agent: 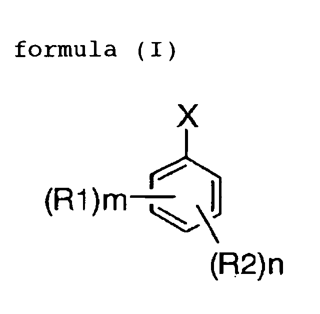 wherein X represents a substituent; R1 represents an electron-withdrawing substituent having a Hammett σₚ constant of more than 0; m represents an integer of 1 to 5; R2 represents a substituent; n represents an integer of 0 to 4; m + n is from 1 to 5; when m is 2 or more, multiple R1s may be the same or different; when n is 2 or more, multiple R2s may be the same or different; R1s, or R2s, or R1 and R2 may bond to each other to form a ring; 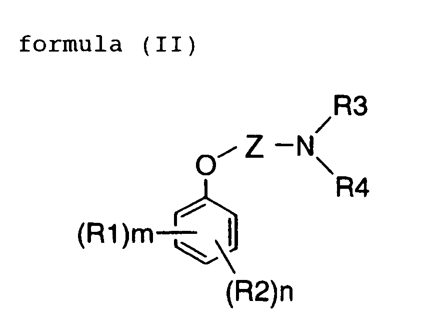 wherein R1 represents an electron-withdrawing substituent having a Hammett σₚ constant of more than 0; m represents an integer of 1 to 5; R2 represents a substituent; n represents an integer of 0 to 4; m + n is from 1 to 5; when m is 2 or more, multiple R1s may be the same or different; when n is 2 or more, multiple R2s may be the same or different; R1s, or R2s, or R1 and R2 may bond to each other to form a ring; R3 and R4 each independently represent an alkyl, aryl, or heterocyclic group; R3 and R4 may bond to each other to form a ring; and Z represents C=O or S(O)p, wherein p is 1 or 2.

2. The process as claimed in claim 1, wherein the compound represented by formula (II) is a compound synthesized by causing a compound represented by the following formula (III) to react with a compound represented by the following formula (IV): 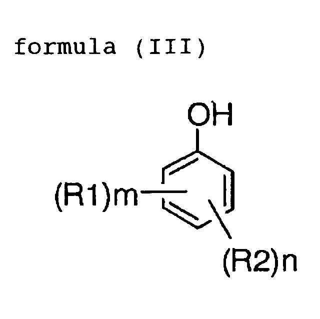 wherein R1 represents an electron-withdrawing substituent having a Hammett σₚ constant of more than 0; m represents an integer of 1 to 5; R2 represents a substituent; n represents an integer of 0 to 4; m + n is from 1 to 5; when m is 2 or more, multiple R1s may be the same or different; when n is 2 or more, multiple R2s may be the same or different; and R1s, or R2s, or R1 and R2 may bond to each other to form a ring; 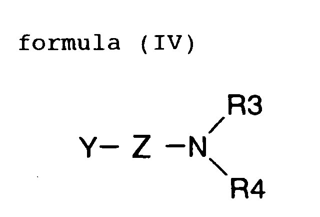 wherein R3 and R4 each independently represent an alkyl, aryl, or heterocyclic group; R3 and R4 may bond to each other to form a ring; Z represents C=O or S(O)p wherein p is 1 or 2; and Y represents a halogen atom.

3. The process as claimed in claim 1 or 2, wherein R1 is a nitro group.

4. The process as claimed in any one of claims 1 to 3, wherein X in formula (I) is a group that bonds to the benzene ring via a nitrogen or sulfur atom.

5. The process as claimed in claim 2, wherein the compound represented by formula (II) and synthesized by causing the compound represented by formula (III) to react with the compound represented by formula (IV) is used, without being isolated, in the form of a reaction mixture.

6. The process as claimed in any one of claims 1 to 5, wherein m in formula (I) is 1.

7. The process as claimed in claim in claims 1 to 6, wherein, in formula (II), Z represents S(O)p and p is 2.

8. The process as claimed in any one of claims 1 to 7, wherein R3 and R4 in formula (II) each are an alkyl group.

9. The process as claimed in claim 2, wherein Y in formula (IV) is a chlorine atom.
